# EUROPÄISCHE PATENTSCHRIFT

(11) **EP 1 465 528 B1**
(45) Veröffentlichungstag und Bekanntmachung des Hinweises auf die Patenterteilung: **31.01.2007**
(21) Anmeldenummer: 02796510.2
(22) Anmeldetag: 16.12.2002
(51) Int. Cl.: A61B 5/145, A61M 5/34

(54) **BLUTENTNAHMEVORRICHTUNG**
BLOOD WITHDRAWAL DEVICE
DISPOSITIF DE PRELEVEMENT DU SANG

(30) Priorität: 21.12.2001 DE 10163716
(43) Veröffentlichungstag der Anmeldung: 13.10.2004
(73) Patentinhaber: Sarstedt AG & Co., 51588 Nümbrecht (DE)
(72) Erfinder: SARSTEDT, Walter, 51588 Nümbrecht (DE)
(74) Vertreter: Grosse, Wolf-Dietrich Rüdiger
(86) Internationale Anmeldenummer: PCT/DE2002/004588
(87) Internationale Veröffentlichungsnummer: WO 2003/055390

(56) Entgegenhaltungen:
- DE-A- 2 903 167
- US-A- 4 378 812
- US-A- 5 755 701

## Beschreibung

Die Erfindung betrifft eine Vorrichtung zur Aufnahme von Körperflüssigkeiten, mit einem Röhrchen, das an seinem vorderen Ende einen Dom mit einem durchstechbaren Stopfen für eine auf den Dom aufsteckbare Führungshülse trägt, die auf der dem Dom zugewandten Seite eine Kanüle mit einem Ventilgummi und auf der dem Dom abgewandten Seite ein Verbindungsstück oder den vorderen Teil einer Doppelkanüle aufweist.

Solche Vorrichtungen werden z.B. benötigt sowohl zur Blutentnahme aus einem Blutgefäß oder aus einem Blutspendebeutel als auch zur Probennahme aus einem Sammelbehälter für beispielsweise Urin. Hierbei tritt in allen Fällen die Schwierigkeit auf, daß das die Kanüle umschließende bzw. einhüllende, sich beim Zusammenfügen bzw. Aufstecken der Führungshülse auf den Dom ziehharmonikaartig zusammenschiebende Ventilgummi eine der Haltekraft zwischen Führungshülse und Dom entgegenwirkende Feder- bzw. Rückstellkraft ausübt, die dazu führen kann, daß sich die Führungshülse von dem Dom der Kappe abdrückt. Um dies zu vermeiden, müssen Gegenmaßnahmen getroffen werden.

Bei einer aus der DE 30 49 503 C bekannten Blutentnahmevorrichtung besitzt die das Entnahmeröhrchen an seinem vorderen Ende abschließende Kappe einen zylindrischen, in axialer Richtung vorstehenden Dom. Der Dom ist an seinem vorderen Ende durch einen durchstechbaren Verschlußstopfen abgeschlossen, der auf einer mit einer Mittelbohrung versehenen Stirnplatte des Doms aufliegt und von einem am vorderen Ende umgebördelten Kragen gehalten wird. Die rohrförmige Führungshülse, die an ihrem vorderen Ende in einem Halter eine doppelendige, beiderseits mit einer Schneidkante versehene Kanüle trägt, deren aus der Führungshülse vorstehendes Ende zur Einführung in eine Vene dient, während ihr hinteres Ende so weit in die Führungshülse hineinragt, daß es beim Ansetzen der Führungshülse an das Entnahmeröhrchen den Verschlußstopfen durchsticht, ist axial verschiebbar und drehbar auf dem Dom angeordnet. Das hintere, in die Führungshülse hineinragende Kanülenende wird von einem sackartigen Schlauch (Ventilgummi) solcher Länge eingehüllt, daß die Schneidkante des hinteren Kanülenendes bei gestrecktem Schlauch noch nicht dessen Boden berührt.

Damit die Führungshülse trotz der Federkraft des Ventilgummis in ihrer Position auf dem Dom gehalten wird, ist zur Verbindung der Doppelkanüle mit dem Dom letzterer mit einem seitlich vorstehenden Haltenocken versehen, dem winkelartige Schlitzausnehmungen in der Führungshülse zugeordnet sind. Mittels des in einen der über den Umfang verteilten Schlitze eingeführten Haltenockens läßt sich eine bajonettverschlußartige Drehverriegelung der in lockerer Passung auf dem Dom sitzenden Führungshülse der Doppelkanüle erreichen. Eine derartige Drehverriegelung gewährleistet einen sehr sicheren Zusammenhalt der zusammengefügten Teile der Blutentnahmevorrichtung, erhöht allerdings deren Herstellungsaufwand. Außerdem setzt das Ankuppeln bzw. Aufsetzen und Verriegeln der Führungshülse voraus, daß der Haltenocken zunächst duch Drehen des Entnahmeröhrchens mit der verschließenden Schraub- oder Stopfenkappe in Flucht mit einem Einführschlitz gebracht werden muß, was eine suchende Handhabung erfordert, weil die Teile gezielt gegeneinander ausgerichtet werden müssen.

Aus der DE 692 25 609 T2 ist ein Schutzgehäuse für eine in einen Kanülenhalter eingeschraubte Kanüle bekannt. Damit sich das Schutzgehäuse um den Halter verdrehen läßt, ist das Schutzgehäuse mit einem innen eine umlaufende Nut aufweisenden Ring formschlüssig auf einer an einem Dom des Halters ausgebildeten Wulst angeordnet.

Der Erfindung liegt die Aufgabe zugrunde, eine Vorrichtung der eingangs genannten Art mit einer einfachen und bedienungssicheren Steckverbindung für die beiden zusammenzufügenden Teile zu schaffen, die einen geringeren Herstellungsaufwand erfordert und bei gleichzeitig einfacherer Handhabung eine Haltekraft zwischen Führungshülse und Dom ermöglicht, die größer ist als die entgegenwirkende Federkraft (Rückstellkraft) des Ventilgummis.

Diese Aufgabe wird mit den im kennzeichnenden Teil von Anspruch 1 angegebenen Merkmalen gelöst.

Die für das Zusammenspiel von nachgiebigem Abschnitt der Führungshülse und Dom zur Erzeugung der gewünschten Haltekraft erfindungsgemäßen Durchmesserverhältnisse der entsprechenden Bereiche am Dom und Führungshülse mit dem Übermaß lassen sich durch Vergrößern des Doms im Verbindungsbereich erreichen, beispielsweise durch eine Wulst,Zylinder, Rippen, Konus, Segmente oder dergleichen. Mit den erfindungsgemäßen Maßnahmen lassen sich gleichzeitig mehrere Vorteile ausschöpfen. Aufgrund der nur begrenzten Länge des nachgiebigen Abschnitts bezogen auf die Gesamtlänge der Führungshülse liegt eine für die notwendige Haftung sorgende Klemmkraft in dem definierten Bereich des endseitigen Abschnitts vor, während eine hinlänglich große Teillänge der Führungshülse unverändert hart und damit unnachgiebig ist, womit ein sich beim Anund Abkoppeln von Entnahmeröhrchen nicht verformender, die Klemmkraft nicht beeinflussender Griffbereich vorliegt. Die Aufweitung durch den partiell größeren Durchmesser des Doms wirkt sich folglich ausschließlich in dem begrenzten Abschnitt außerhalb des Griffbereiches aus. Die nurmehr partielle Aufweitung der Führungshülse ermöglicht es, die erforderliche Haftung präziser zu bestimmen und festzulegen, wobei die kraftschlüssige Verbindung größer als die Druckkraft des Ventilgummis ist, was ein Abdrücken der Führungshülse von dem Dom verhindert. Trotz der relativ kleinen Durchmesser, die die Steckverbinderteile (Führungshülse und Dom) aufweisen, läßt sich der gewünschte selbstklemmende Halt erreichen, wobei sich die Teile gleichzeitig aber leichter und ruckfreier zusammenstecken und lösen lassen als eine herkömmliche Luer-Konus-Verbindung. Eine suchende Handhabung entfällt, da eine gezielte Ausrichtung nicht erforderlich ist.

Um die Nachgiebigkeit der Führungshülse auf den Bereich außerhalb des harten Griffbereiches festzulegen, läßt sich beispielsweise bei gegenüber der übrigen Führungshülse unveränderter Wanddicke am Vorderende der Hülse vorzugsweise mindestens ein Längsschlitz vorsehen, dessen Tiefe im wesentlichen die Länge des nachgiebigen Abschnitts definiert. Der bzw. die Schlitze bewirken, daß das ansonsten verformungssteife Material einerseits die notwendige Flexibilität für eine einfache Handhabung aufweist und andererseits die notwendige Haltekraft (kraftschlüssige Verbindung) gegen die Federkraft des Ventilgummis gewährleistet.

Alternativ könnte anstelle der Schlitze der nachgiebige Abschnitt so dünnwandig und/oder vom Material her so ausgelegt werden, daß sich die gewünschte Haltekraft ergibt. Wenn der Dom mit einem den Domaußendurchmesser über den Innendurchmesser der Führungshülse hinaus vergrößernden Ring, einer Außenwulst oder mit einem Konusabschnitt ausgebildet wird, ist bei der Herstellung des Doms bzw. einer einen Dom besitzenden Kappe eine spritzgießtechnische Herstellung ohne aufwendige Schieberwerkzeuge möglich. Aber auch mit z. B. im gleichen Abstand über dem Umfang des Doms verteilt angeordneten Nocken oder auch nur einer Nocke würde der Abschnitt der Führungshülse beim Aufstecken zum haftenden Halt aufgeweitet, so daß die Führungshülse eine kraftschlüssige Verbindung mit dem Dom eingeht und damit eine kontrollierte Vorspannung bzw. Haltekraft vorliegt.

Weitere Merkmale und Einzelheiten der Erfindung ergeben sich aus dem Patentanspruch und der nachfolgenden Beschreibung von in den Zeichnungen anhand einer Vorrichtung zur Entnahme von Blut dargestellten Ausführungsbeispielen des Gegenstandes der Erfindung. Es zeigen:
- Fig. 1: ein Entnahmeröhrchen mit Führungshülse, explosiv dargestellt;
- Fig. 2: eine Gesamtansicht der auf einen Dom des Entnahmeröhrchens nach Fig. 1 aufgesteckten Führungshülse;
- Fig. 3: eine andere Ausführung von Entnahmeröhrchen und Führungshülse, explosiv dargestellt;
- Fig. 4: als Gesamtansicht die auf einen Dom des Entnahmeröhrchens aufgesteckte Führungshülse nach Fig. 3;
- Fig. 5: einen Schnitt entlang der Linie V-V durch die Führungshülse nach Fig. 3;
- Fig. 6: eine weitere Ausführung eines Entnahmeröhrchens und einer Führungshülse, explosiv dargestellt;
- Fig. 7: als Gesamtansicht die auf einen Dom des Entnahmeröhrchens aufgesteckte Führungshülse nach Fig. 6; und
- Fig. 8: einen Schnitt entlang der Linie VIII-VIII durch die Führungshülse nach Fig. 6.

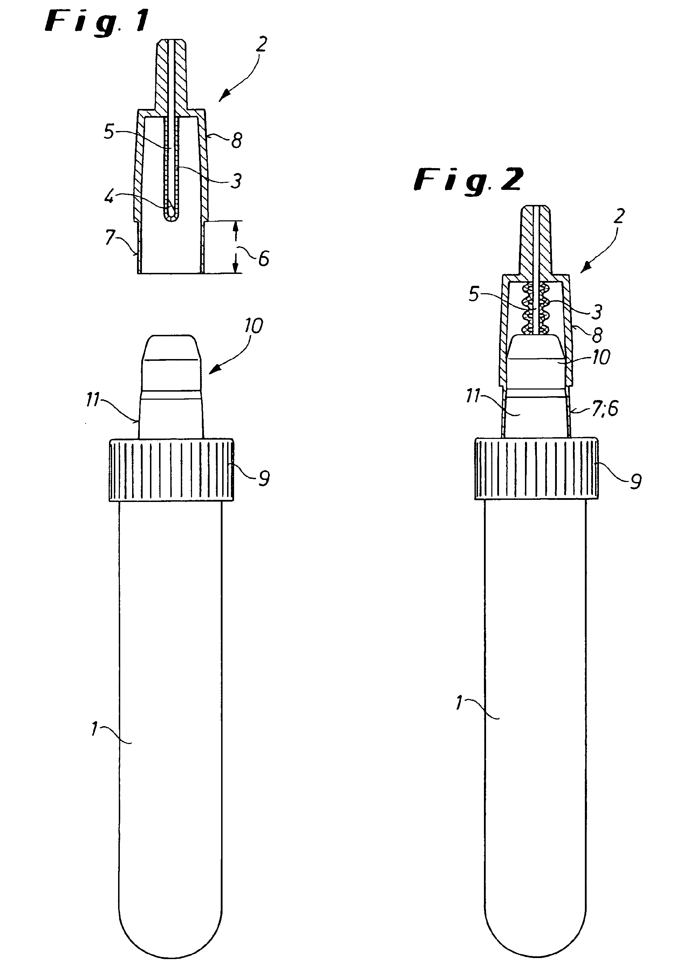

Eine Blutentnahmevorrichtung nach den Fig. 1 und 2 umfaßt ein Entnahmeröhrchen 1 mit einem Dom 10 und eine - in allen Ausführungen beispielsweise mit einem Luer-Konus dargestellt - Führungshülse 2, die eine von einem Ventilgummi 3 umschlossene, eine angeschärfte Schneidkante 4 aufweisende Kanüle 5 aufnimmt. Bei anderen Varianten einer Führungshülse ist auf der von dem Dom abgewandten Seite ein Verbindungsstück oder der vordere Teil einer Doppelkanüle vorgesehen. Das dem Entnahmeröhrchen 1 zugewandte Ende der Führungshülse 2 ist mit einem nachgiebigen Abschnitt 6 ausgebildet, gemäß den Fig. 1 und 2 in Form eines in seiner Wanddicke gegenüber der übrigen Führungshülse 2 reduzierten Hülsenendringes 7. Oberhalb dieses Abschnittes 6 besteht die Führungshülse 2 unverändert aus einem verformungssteifen Kunststoffmaterial und stellt dort somit einen für die Handhabung beim An- und Abkoppeln von Entnahmeröhrchen äußerst vorteilhaften, unverformbaren Griffbereich 8 bereit.

Das Entnahmeröhrchen 1 ist oben mit einer Kappe 9 verschlossen. Diese besitzt einen Dom 10, der zum Domfuß hin, etwa beginnend ab der Hälfte der Domlänge, ein Durchmesser-Übermaß aufgrund einer gegenüber dem Innendurchmesser der Führungshülse größeren Erhöhung in Form eines Konusabschnitts 11 aufweist. Im funktionsbereiten Zustand der Blutentnahmevorrichtung befindet sich die Führungshülse 2 auf dem Dom 10 des Entnahmeröhrchens 1, wie in Fig. 2 dargestellt. Beim Aufstecken der Führungshülse 2 auf den Dom 10 durchdringt die Kanüle 5 mit ihrer Schneidkante 4 einen in der Kappe 9 angeordneten, nicht gezeigten Stopfen, bis die Schneidkante 4 frei im Inneren des Entnahmeröhrchens 1 liegt; das dabei zuvor von der Schneidkante 4 natürlich ebenfalls durchdrungene Ventilgummi 3 schiebt sich hierbei zieharmonikaartig zusammen, wie in Fig. 2 zu erkennen. Der Halt bzw. die Haftung der Führungshülse 2 auf dem Dom 10 wird gegen die Feder- bzw. Rückstellkraft des Ventilgummis 3 durch den den nachgiebigen Abschnitt 6 der Führungshülse 2 aufweitenden Konusabschnitt 11 erreicht, der den das Übermaß aufweisenden Dom 10 in diesem Verbindungsbereich mit der gewünschten Haltekraft kraftschlüssig umschließt.

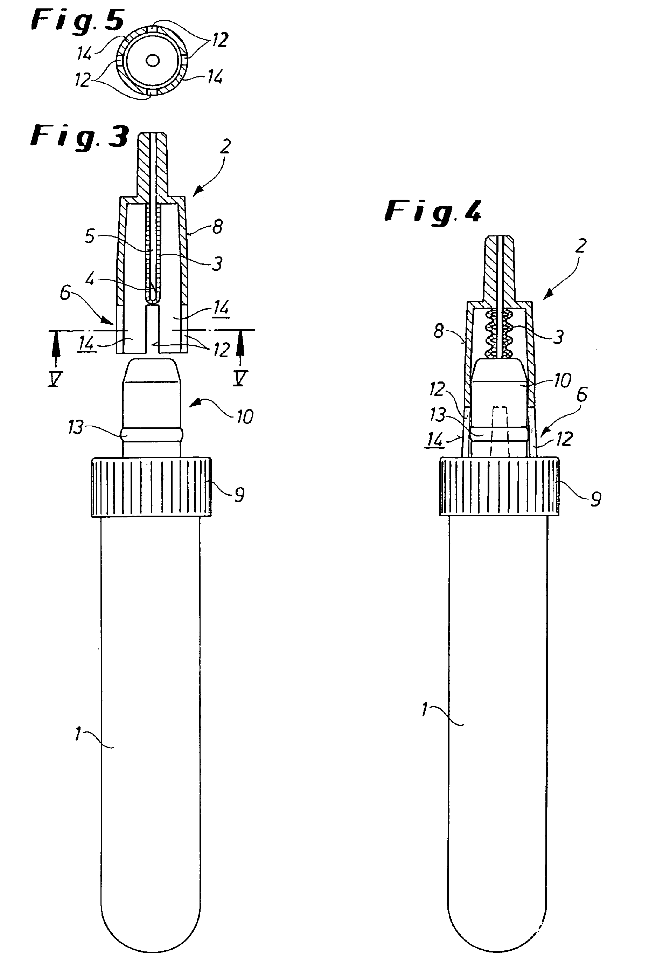

Die Ausführungen von Blutentnahmevorrichtungen nach den Fig. 3 bis 5 bzw. 6 bis 8 unterscheiden sich lediglich durch konstruktive Änderungen an dem nachgiebigen Abschnitt der Führungshülse bzw. dem Dom der Kappe des Entnahmeröhrchens gegenüber den Figuren 1 und 2, so daß übereinstimmende Bauteile mit denselben Bezugsziffern versehen sind; gemeinsam ist hingegen allen Ausführungen, daß ein Durchmesser-Übermaß des Domes den nachgiebigen Abschnitt der Führungshülse aufweitet, so daß die Führungshülse kraftschlüssig mit dem Dom verbunden wird.

Bei der Blutentnahmevorrichtung nach den Fig. 3 bis 5 wird die Nachgiebigkeit des vor dem Griffbereich 8 liegenden Abschnitts 6 durch im gleichen Teilungsabstand angeordnete, im Ausführungsbeispiel vier, Längsschlitze 12 (vgl. Fig. 5) erreicht, die zwischen sich nachgiebige Flügel 14 einschließen bzw. begrenzen. Der Dom 10 besitzt hier eine Erhöhung in Form eines den Domaußendurchmesser vergrößernden Ringes bzw. einer Außenwulst 13, die den durch die Schlitze 12 nachgiebigen bzw. weichgemachten Abschnitt 6 bzw. dessen Flügel 14 beim Aufstecken der Führungshülse 2 auf den Dom 10 aufweitet (vgl. Fig. 4), wodurch die Führungshülse 2 auf dem Entnahmeröhrchen 1 kraftschlüssig mit einer Haltekraft festgehalten wird, die größer ist als die entgegenwirkende Rückstellkraft des Ventilgummis 3.

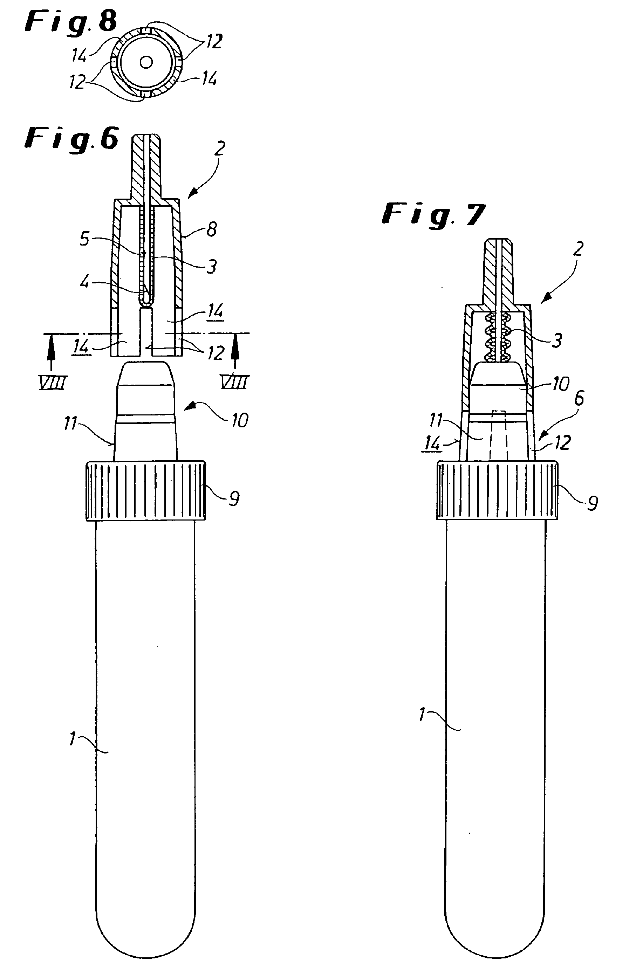

Die Variante einer Blutentnahmevorrichtung nach den Fig. 6 bis 8 besitzt ebenfalls eine Führungshülse 2 mit Längsschlitzen 12 und von diesen im Abschnitt 6 begrenzten Flügeln 14, wie zuvor anhand der Fig. 3 bis 5 beschrieben. Zur Aufweitung des Abschnitts 6 beim Aufstecken der Führungshülse 2 auf den Dom 10 der Kappe 9 des Entnahmeröhrchens 1 weist der Dom hier entsprechend der Ausführung nach den Fig. 1 und 2 einen zum Fußende des Doms hin ausgebildeten Konusabschnitt 11 auf.

Unabhängig davon, wie im Einzelfall die dem Abschnitt 6 der Führungshülse 2 am Dom 10 der Kappe 9 zur Schaffung des Durchmesser-Übermaßes zugeordnete Erhöhung konkret ausgestaltet ist, so wird doch in jedem Fall aufgrund des damit gegenüber dem Innendurchmesser der Führungshülse 2 vorliegenden, im Verbindungsbereich partiell größeren Außendurchmessers des Doms für die Führungshülse 2 über den sich aufweitenden Abschnitt 6 die zum sicheren Halt benötigte Klemmkraft erreicht. Dies auch dann, wenn statt einer eher flächigen Erhöhung, wie bei einem Gegenkonus oder einer Außenwulst, lediglich punktförmige Erhöhungen vorgesehen sind. Zu erwähnen ist weiterhin auch, daß die Nachgiebigkeit des Abschnitts 6 in günstiger Weise weiterhin eventuelle Fertigungstoleranzen ausgleicht bzw. überbrückt, was die Herstellung wesentlich günstiger gestaltet.

## Patentansprüche

1. Vorrichtung zur Aufnahme von Körperflüssigkeiten, mit einem Entnahmeröhrchen (1), das an seinem vorderen Ende einen Dom (10) mit einem durchstechbaren Stopfen für eine auf den Dom (10) aufsteckbare Führungshülse (2) trägt, die auf der dem Dom (10) zugewandten Seite eine Kanüle (5) mit einem Ventilgummi (3) und auf der dem Dom abgewandten Seite ein Verbindungsstück oder den vorderen Teil einer Doppelkanüle aufweist.
**dadurch gekennzeichnet,**
**daß** die Führungshülse (2) aus einem verformungssteifen Material besteht und an ihrem vorderen, außerhalb eines von der normalen Handhabung vorgegebenen Griffbereichs (8) liegenden Ende mit einem nachgiebigen Abschnitt (6) ausgebildet ist, der Dom (10) in Relation zum Innendurchmesser der Führungshülse (2) in einem Verbindungsbereich ein Durchmesser-Übermaß aufweist, wobei der größere Domdurchmesser in der zusammengefügten Gebrauchslage den nachgiebigen Abschnitt (6) aufweitet, der den Verbindungsbereich des Doms (10) zur Erzielung der Haltekraft kraftschlüssig umschließt.

## Claims

1. A device for receiving body fluids, with a withdrawal tube (1) that has on its distal end a dome (10) with a puncturable stopper for a guide sleeve (2) that can be stuck onto the dome (10), said guide sleeve having on the side facing the dome (10) a cannula (5) with a rubber valve (3) and on the side facing away from the dome a connecting piece or the distal part of a double cannula,
**characterized in that**
the guide sleeve (2) is made of a deformationally rigid material and is designed with a flexible section (6) on its distal end, which is outside of a handle area (8) that is determined by normal handling; the dome (10) has an excess diameter in relation to the inside diameter of the guide sleeve (2) in a connecting area, whereby the greater dome diameter has a flexible section (6) surrounding in a frictionally engaged manner the connecting area of the dome (10) to achieve the holding force in the assembled and ready-to-use position.

## Revendications

1. Dispositif destiné à recevoir des liquides physiologiques, comportant un petit tube de prélèvement (1) qui présente à son extrémité antérieure un mandrin (10) comportant un bouchon pouvant être percé pour un manchon de guidage (2) pouvant être fiché sur le mandrin (10) présentant, de son côté tourné vers le mandrin (10), une canule (5) avec un caoutchouc de vanne (3) et, de son côté détourné du mandrin, une pièce de raccordement ou la partie antérieure d'une double canule,
**caractérisé en ce que**
le manchon de guidage (2) est composé d'un matériau rigide à la déformation et est réalisé, à son extrémité antérieure se trouvant en dehors d'une zone de préhension (8) prescrite par la manipulation normale, avec une section souple (6), que le mandrin (10) présente, par rapport au diamètre intérieur du manchon de guidage (2), dans une zone de raccordement, une surcote de diamètre, alors que le plus grand diamètre de mandrin, en position d'utilisation assemblée, élargit la section souple (6) qui entoure par correspondance mécanique la zone de raccordement du mandrin (10) pour obtenir la force de retenue.
